# EUROPEAN PATENT APPLICATION

(11) **EP 4 473 969 A1**
(43) Date of publication of application: **11.12.2024**
(21) Application number: 23749862.1
(22) Date of filing: 03.02.2023
(51) Int. Cl.: A61K 31/519, A61K 9/20, A61K 9/30, A61K 9/32, A61K 9/36, A61K 47/26, A61K 47/38, A61P 5/24

(54) **ORAL SOLID PREPARATION**

(30) Priority: 04.02.2022 JP 2022016505
(71) Applicant: Kissei Pharmaceutical Co., Ltd., Matsumoto-Shi, Nagano 399-8710 (JP)
(72) Inventor: IMAMURA, Yusuke, Azumino-shi, Nagano 399-8304 (JP); MIMURA, Kazuki, Azumino-shi, Nagano 399-8304 (JP)
(74) Representative: Boult Wade Tennant LLP
(86) International application number: PCT/JP2023/003575
(87) International publication number: WO 2023/149546

(57) **Abstract**

An object of the present invention is to provide an oral solid preparation having a rapid dissolution property even when the content of Linzagolix or a pharmacologically acceptable salt thereof in the oral solid preparation varies over a wide range.

The present invention relates to an oral solid preparation comprising Linzagolix or a pharmacologically acceptable salt thereof; crystalline cellulose; low-substituted hydroxypropyl cellulose; and one or more disintegrants selected from the group consisting of carmellose sodium, carmellose calcium and croscarmellose sodium, and the like.

## Description

### Technical Field

The present invention relates to an oral solid preparation containing a fused heterocyclic compound or a pharmacologically acceptable salt thereof. More particularly, the present invention relates to an oral solid preparation containing, as an active ingredient, a compound represented by the following formula (chemical name: 3-[2-fluoro-5-(2,3-difluoro-6-methoxybenzyloxy)-4-methoxyphenyl]-2,4-dioxo-1,2,3,4-tetrahydrothieno[3,4-d]pyrimidine-5-carboxylic acid, hereinafter referred to as "Linzagolix") or a pharmacologically acceptable salt thereof, which has a gonadotropin-releasing hormone antagonistic activity and is useful as a preventive or therapeutic agent for sex hormone-dependent diseases such as prostatic hyperplasia, uterine fibroids, endometriosis, metrofibroma, precocious puberty, amenorrhea, premenstrual syndrome and dysmenorrhea.

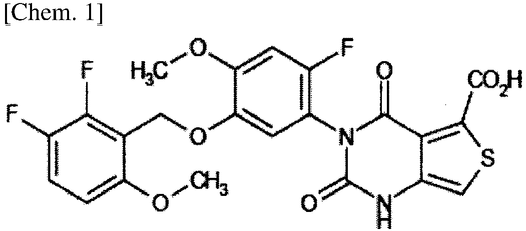

Particularly preferably, the present invention relates to an oral solid preparation containing a compound represented by the following formula (chemical name: 3-[2-fluoro-5-(2,3-difluoro-6-methoxybenzyloxy)-4-methoxyphenyl]-2,4-dioxo-1,2,3,4-tetrahydrothieno[3,4-d]pyrimidine-5-carboxylic acid choline salt, hereinafter referred to as "Linzagolix choline salt") as an active ingredient.

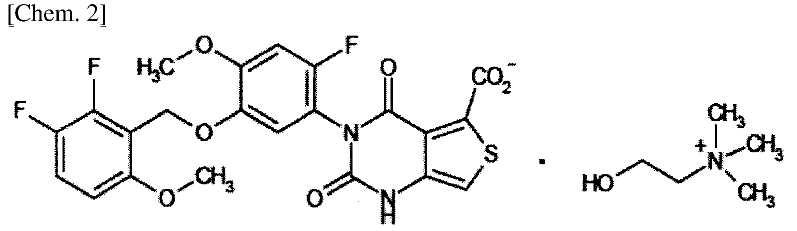

### Background Art

It is known that Linzagolix has a gonadotropin-releasing hormone antagonistic activity and is useful as a preventive or therapeutic agent for sex hormone-dependent diseases such as prostatic hyperplasia, uterine fibroids, endometriosis, metrofibroma, precocious puberty, amenorrhea, premenstrual syndrome and dysmenorrhea (see, for example, Patent literatures 1 and 2).

With respect to pharmaceutical compositions containing Linzagolix or a pharmacologically acceptable salt thereof as an active ingredient, oral dosage forms as administration forms of pharmaceutical compositions have been exemplified as general descriptions targeting fused heterocyclic derivatives described by general formulae including Linzagolix, and it has been reported that pharmaceutical compositions can be prepared by mixing with commonly used pharmaceutical carriers (see, for example, Patent literature 1).

However, the general description of Patent literature 1 does not give a suggestion for selecting a specific pharmaceutical formulation of an oral solid preparation containing Linzagolix or a pharmacologically acceptable salt thereof having a rapid dissolution property as an active ingredient.

Patent literature 1: International Publication WO2007/046392 pamphlet
Patent literature 2: International Publication WO2011/099507 pamphlet

### SUMMARY OF THE INVENTION

### Problems to be Solved by the Invention

The present inventors have studied to develop a pharmaceutical formulation having a rapid dissolution property even when the content of the active ingredient varies over a wide range, and have found that when the content of Linzagolix or a pharmacologically acceptable salt thereof in the oral solid preparation is high (for example, 60% by mass), the dissolution property is lowered and the rapid dissolution property cannot be satisfied.

An object of the present invention is to provide an oral solid preparation having a rapid dissolution property even when the content of Linzagolix or a pharmacologically acceptable salt thereof in the oral solid preparation varies over a wide range.

Another object of the present invention is to provide an oral solid preparation containing Linzagolix or a pharmacologically acceptable salt thereof, which has good storage stability.

### Means for Solving the Problems

As a result of intensive studies to solve the above problems, the present inventors have found that the above problems can be solved by the following means, and have completed the present invention.

That is, the present invention relates to the following [1] to [18] and the like.
[1] An oral solid preparation comprising the following 1) to 4):
   1) Linzagolix or a pharmacologically acceptable salt thereof;
   2) crystalline cellulose;
   3) low-substituted hydroxypropyl cellulose; and
   4) one or more disintegrants selected from the group consisting of carmellose sodium, carmellose calcium and croscarmellose sodium.
[2] The oral solid preparation described in the above-mentioned [1], which comprises the following A) and B):
   A) a granulated material containing the following 1) to 3):
      1) Linzagolix or a pharmacologically acceptable salt thereof;
      2) crystalline cellulose; and
      3) low-substituted hydroxypropyl cellulose; and
   B) one or more disintegrants selected from the group consisting of carmellose sodium, carmellose calcium and croscarmellose sodium.
[3] The oral solid preparation described in the above-mentioned [1] or [2], wherein Linzagolix or a pharmacologically acceptable salt thereof is Linzagolix choline salt.
[4] The oral solid preparation described in any one of the above-mentioned [1]-[3], wherein the dissolution ratio of the oral solid preparation in a pH 6.8 test solution or water after 15 minutes is 80% or more.
[5] The oral solid preparation described in any one of the above-mentioned [1]-[4], wherein the total amount of the low-substituted hydroxypropyl cellulose and one or more disintegrants selected from the group consisting of carmellose sodium, carmellose calcium and croscarmellose sodium in the oral solid preparation is 20% by mass or less.
[6] The oral solid preparation described in any one of the above-mentioned [1]-[5], which comprises a binder.
[7] The oral solid preparation described in the above-mentioned [6], wherein the binder is one or more selected from the group consisting of hydroxypropyl cellulose, hypromellose, polyvinylpyrrolidone, polyvinyl alcohol and polyethylene glycol.
[8] The oral solid preparation described in the above-mentioned [6] or [7], wherein the content of the binder in the oral solid preparation is 5% by mass or less.
[9] The oral solid preparation described in any one of the above-mentioned [1]-[8], wherein the content of Linzagolix or a pharmacologically acceptable salt thereof in the oral solid preparation is 15-65% by mass.
[10] The oral solid preparation described in any one of the above-mentioned [1]-[9], wherein the content of crystalline cellulose in the oral solid preparation is 15% by mass or more.
[11] The oral solid preparation described in any one of the above-mentioned [1]-[10], which comprises an excipient.
[12] The oral solid preparation described in the above-mentioned [11], wherein the excipient is one or more selected from the group consisting of lactose hydrate and D-mannitol.
[13] The oral solid preparation described in any one of the above-mentioned [1]-[12], wherein the amount of Linzagolix or a pharmacologically acceptable salt thereof per oral solid preparation is 75 mg, 100 mg or 200 mg in terms of Linzagolix.
[14] The oral solid preparation described in any one of the above-mentioned [1]-[13], wherein the oral solid preparation is a tablet.
[15] The oral solid preparation described in the above-mentioned [14], wherein the tablet is a film-coated tablet.
[16] The oral solid preparation described in the above-mentioned [15], wherein the film-coated tablet is a film-coated tablet film-coated with one or more coating agents selected from the group consisting of hypromellose and a polyvinyl alcohol-polyethylene glycol graft copolymer.
[17] The oral solid preparation described in any one of the above-mentioned [1]-[16], which is an oval tablet having a longest dimension of 20 mm or less.
[18] The oral solid preparation described in any one of the above-mentioned [1]-[16], which is a circular tablet having a tablet diameter of 13 mm or less.

### Effects of the Invention

The oral solid preparation of the present invention has a rapid dissolution property even when the content of Linzagolix or a pharmacologically acceptable salt thereof varies over a wide range.

In addition, the oral solid preparation of the present invention has good storage stability.

### DETAILED DESCRIPTION OF THE INVENTION

Hereinafter, embodiments of the present invention will be described in more detail. In the present invention, each term has the following meaning unless otherwise specified.

As Linzagolix or a pharmacologically acceptable salt thereof used in the present invention, a commercially available one may be used, or it may be produced by a method described in literatures (see, for example, Patent literature 1) or a method analogous thereto.

In the present invention, "Linzagolix or a pharmacologically acceptable salt thereof" is preferably Linzagolix choline salt.

Linzagolix or a pharmacologically acceptable salt thereof used in the present invention is, for example, in the form of particles having no aggregated mass, and may be crushed, pulverized, or the like as necessary. The average particle diameter (50% particle diameter, D50) of Linzagolix or a pharmacologically acceptable salt thereof is, for example, 50 µm or less, and preferably 40 µm or less.

In one embodiment, the average particle diameter of Linzagolix or a pharmacologically acceptable salt thereof is 1-40 µm, more preferably 1-30 µm.

In the present invention, the amount of Linzagolix or a pharmacologically acceptable salt thereof in the oral solid preparation is 25 to 200 mg, preferably 50 to 200 mg, and more preferably 75 to 200 mg, in terms of Linzagolix.

In one embodiment, the amount of Linzagolix or a pharmacologically acceptable salt thereof in the oral solid preparation is 25, 50, 75, 100, 125, 150, 175 or 200 mg, preferably 50, 75, 100 or 200 mg in terms of Linzagolix.

In the present invention, the content of Linzagolix or a pharmacologically acceptable salt thereof in the oral solid preparation is, for example, 10-65% by mass, 15-65% by mass, 15-60% by mass or 20-60% by mass.

In one embodiment, the content of Linzagolix or a pharmacologically acceptable salt thereof in the oral solid preparation is preferably 25-65% by mass, more preferably 30-60% by mass.

In one embodiment, the oral solid preparation of the present invention contains 15 to 60% by mass of Linzagolix choline salt per oral solid preparation.

In one embodiment, the oral solid preparation is a tablet containing Linzagolix or a pharmacologically acceptable salt thereof in an amount of 25, 50, 75, 100, 125, 150, 175 or 200 mg, preferably 50, 75, 100 or 200 mg in terms of Linzagolix, and having a tablet diameter of 10 mm or less, preferably 8 mm or less, more preferably 6 mm or less.

In one embodiment, the oral solid preparation is a tablet containing Linzagolix or a pharmacologically acceptable salt thereof in an amount of 50, 75, 100 or 200 mg in terms of Linzagolix and having a tablet diameter of 10 mm or less.

In the present invention, "a wide range of the content of the active ingredient" means that the content of Linzagolix or a pharmacologically acceptable salt thereof in the oral solid preparation is, for example, 15 to 60% by mass.

The oral solid preparation of the present invention can also be used in combination with another drug. When the oral solid preparation of the present invention is used in combination with another drug, their administration timing is not limited, and they may be administered simultaneously or at intervals.

In addition, the oral solid preparation of the present invention and another drug may be administered as separate preparations, or may be administered as a single preparation containing the oral solid preparation of the present invention and another drug.

In one embodiment, the oral solid preparation of the present invention may contain one or more other drugs in addition to Linzagolix or a pharmacologically acceptable salt thereof.

Examples of the "another drug" used in the present invention include estrogen (for example, estradiol) and progestin (for example, norethindrone acetate).

The "low-substituted hydroxypropyl cellulose" used in the present invention is not particularly limited as long as it is used as a pharmaceutical additive, and one or two or more kinds thereof may be used.

In the present invention, the "low-substituted hydroxypropyl cellulose " means low-substituted hydroxypropyl cellulose containing 5.0 to 16.0% (dry weight) of hydroxypropoxy groups after drying. Preferably, the low-substituted hydroxypropyl cellulose contains 8-14% (dry weight), more preferably 11% (dry weight) of hydroxypropoxy groups.

Examples of the low-substituted hydroxypropyl cellulose include LH-11, LH-21, LH-31, LH-22, and LH-32 (manufactured by Shin-Etsu Chemical Co., Ltd.).

In the present invention, the low-substituted hydroxypropyl cellulose is preferably LH-11, LH-21 or LH-31, more preferably LH-21 or LH-31, and most preferably LH-21.

In the present invention, the content of the low-substituted hydroxypropyl cellulose in the oral solid preparation is preferably 5-20% by mass, more preferably 5-15% by mass, most preferably 5-10% by mass.

In one embodiment, the content of the low-substituted hydroxypropyl cellulose in the oral solid preparation is preferably 5% by mass, 10% by mass, 15% by mass or 20% by mass.

The "crystalline cellulose" used in the present invention is not particularly limited as long as it is used as a pharmaceutical additive, and one or more kinds thereof may be used.

Examples of the crystalline cellulose include CEOLUS (registered trademark) PH-101 and CEOLUS (registered trademark) PH-301 (manufactured by Asahi Kasei Chemicals Corporation).

In the present invention, crystalline cellulose is preferably CEOLUS (registered trademark) PH-101 (manufactured by Asahi Kasei Chemicals Corporation).

In the present invention, the content of crystalline cellulose in the oral solid preparation is preferably 15-30% by mass, more preferably 15-25% by mass.

In one embodiment, the content of crystalline cellulose in the oral solid preparation is preferably 15% by mass, 20% by mass, 25% by mass or 30% by mass.

The "carmellose sodium, carmellose calcium and croscarmellose sodium" used in the present invention are not particularly limited as long as they are used as pharmaceutical additives, and one or two or more kinds thereof may be used.

In the present invention, the "one or more disintegrants selected from the group consisting of carmellose sodium, carmellose calcium, and croscarmellose sodium" may be added either inside a granulated material or outside a granulated material as a component to be added later.

In one embodiment, one or more disintegrants selected from the group consisting of carmellose sodium, carmellose calcium and croscarmellose sodium are preferably added outside a granulated material as a component to be added later other than the granulated material containing Linzagolix or a pharmacologically acceptable salt thereof.

In the present invention, the "component to be added later" means an additive to be added outside a granulated material.

In one embodiment, in the oral solid preparation of the present invention, one or more disintegrants selected from the group consisting of carmellose sodium, carmellose calcium and croscarmellose sodium are preferably one or more disintegrants selected from the group consisting of carmellose calcium and croscarmellose sodium.

In the present invention, the content of one or more disintegrants selected from the group consisting of carmellose sodium, carmellose calcium and croscarmellose sodium in the oral solid preparation is preferably 5-15% by mass, more preferably 5-10% by mass.

In the present invention, the total amount of "low-substituted hydroxypropyl cellulose" and "one or more disintegrants selected from the group consisting of carmellose sodium, carmellose calcium and croscarmellose sodium" in the oral solid preparation is, for example, 25% by mass or less, 20% by mass or less, 15% by mass or less or 10% by mass or less.

In the present invention, the total amount of "low-substituted hydroxypropyl cellulose" and "one or more disintegrants selected from the group consisting of carmellose sodium, carmellose calcium and croscarmellose sodium" in the oral solid preparation is 10 to 25% by mass, preferably 10 to 20% by mass, more preferably 10 to 15% by mass.

In one embodiment, examples of the combination of "low-substituted hydroxypropyl cellulose" and "one or more disintegrants selected from the group consisting of carmellose sodium, carmellose calcium and croscarmellose sodium" in the oral solid preparation of the present invention include a combination of low-substituted hydroxypropyl cellulose and croscarmellose sodium, and a combination of low-substituted hydroxypropyl cellulose and carmellose calcium. A preferred combination is a combination of low-substituted hydroxypropyl cellulose and croscarmellose sodium.

In the present invention, "% by mass" means % by mass of a component in the oral solid preparation. In the case where film coating is applied, "% by mass" was calculated by excluding the mass of the film coated portion.

In the present invention, the "average particle diameter" means a 50% particle diameter (mass-based median diameter, D50). The 50% particle diameter can be measured by a wet method using a laser diffraction-type particle size distribution measuring apparatus (for example, LMS-2000e, manufactured by Seishin Enterprise Co., Ltd.).

In the present invention, the "rapid dissolution property" means that the dissolution ratio at 15 minutes is 80% or more, 85% or more, 90% or more, or 95% or more when the oral solid preparation is subjected to a dissolution test in accordance with Japanese Pharmacopoeia Dissolution Test Method 2, for example, under the conditions of Test Example 1 or Test Example 2. In addition, in one embodiment, the dissolution ratio at 30 minutes is preferably 90% or more, 95% or more, 97% or more, or 99% or more.

In the present invention, "good storage stability" means that the amount of total analogs derived from Linzagolix is 1% or less when the oral solid preparation is stored under given storage conditions.

In one embodiment, the oral solid preparation of the present invention has a rapid dissolution property even when stored (for example, for 6 months) under open conditions at a temperature of 40°C and a relative humidity of 75%, and also has good storage stability since there is no increase in total analogs over time. For example, the storage stability can be evaluated by the method described in Test Example 3.

In one embodiment, the oral solid preparation of the present invention has a rapid dissolution property even when the content of Linzagolix or a pharmacologically acceptable salt thereof is high (for example, 50% by mass or more). Therefore, the oral solid preparation can be miniaturized, and improvement in patient dosage compliance can also be expected.

In one embodiment, the content of Linzagolix or a pharmacologically acceptable salt thereof in the oral solid preparation is preferably 50 to 65% by mass, more preferably 55 to 65% by mass.

The oral solid preparation of the present invention is preferably the following preparations.

### [Oral Solid Preparation 1]

An oral solid preparation comprising the following (1) to (7):

| | | |
|---|---|---|
| (1) | Linzagolix or a pharmacologically acceptable salt thereof | 15 to 60% by mass, |
| (2) | low-substituted hydroxypropyl cellulose | 5 to 10% by mass, |
| (3) | crystalline cellulose | 15 to 30% by mass, |
| (4) | an excipient (preferably, mannitol or lactose hydrate) | 0 to 60% by mass, |
| (5) | a binder (preferably hydroxypropyl cellulose) | 1 to 4% by mass, |
| (6) | a disintegrant (preferably croscarmellose sodium or carmellose calcium) | 5 to 10% |
| | by mass, and | |
| (7) | a lubricant (preferably, magnesium stearate) | 1 to 3% by mass. |

### [Oral Solid Preparation 2]

An oral solid preparation comprising the following (1) to (7):

| | | |
|---|---|---|
| (1) | Linzagolix or a pharmacologically acceptable salt thereof | 15 to 60% by mass, |
| (2) | low-substituted hydroxypropyl cellulose | 5 to 10% by mass, |
| (3) | crystalline cellulose | 15 to 30% by mass, |
| (4) | lactose hydrate | 1 to 30% by mass, |
| (5) | hydroxypropyl cellulose | 1 to 4% by mass, |
| (6) | croscarmellose sodium | 5 to 10% by mass, and |
| (7) | magnesium stearate | 1.5 to 2% by mass. |

### [Oral Solid Preparation 3]

An oral solid preparation comprising the following (1) to (7):

| | | |
|---|---|---|
| (1) | Linzagolix or a pharmacologically acceptable salt thereof | 15 to 60% by mass, |
| (2) | low-substituted hydroxypropyl cellulose | 5 to 10% by mass, |
| (3) | crystalline cellulose | 15 to 30% by mass, |
| (4) | lactose hydrate | 1 to 30% by mass, |
| (5) | hydroxypropyl cellulose | 1 to 4% by mass, |
| (6) | carmellose calcium | 5 to 10% by mass, and |
| (7) | magnesium stearate | 1.5 to 2% by mass. |

In one embodiment, the present invention is the following tablet.

A tablet comprising the following 1) to 3):
1) 15 to 60% by mass of Linzagolix or a pharmacologically acceptable salt thereof;
2) crystalline cellulose; and
3) 10 to 20% by mass of a disintegrant;
the disintegrant is a combination of at least two or more disintegrants, and the % by mass of the disintegrant is the total amount of two or more disintegrants.

In one embodiment, the present invention is the following tablet.

A tablet comprising the following 1) to 4):
1) 15 to 60% by mass of Linzagolix or a pharmacologically acceptable salt thereof;
2) 15 to 25% by mass of crystalline cellulose;
3) 5 to 10% by mass of low-substituted hydroxypropyl cellulose; and
4) 5% by mass of croscarmellose sodium.

In one embodiment, the present invention is the following tablet.

A tablet comprising the following 1) to 4)
1) 55 to 65% by mass of Linzagolix or a pharmacologically acceptable salt thereof;
2) 15 to 25% by mass of crystalline cellulose;
3) 5 to 10% by mass of low-substituted hydroxypropyl cellulose; and
4) 5% by mass of croscarmellose sodium.

In one embodiment, the present invention is the following tablet.

A tablet comprising the following A) and B):
A) a granulated material containing the following 1) to 3):
   1) Linzagolix or a pharmacologically acceptable salt thereof having an average particle diameter of 40 µm or less;
   2) crystalline cellulose; and
   3) low-substituted hydroxypropyl cellulose; and
B) croscarmellose sodium.

In one embodiment, the present invention is the following tablet.

A tablet comprising the following A) to C):
A) a granulated material containing the following 1) to 3):
   1) 15 to 60% by mass of Linzagolix or a pharmacologically acceptable salt thereof;
   2) crystalline cellulose; and
   3) low-substituted hydroxypropyl cellulose,
B) croscarmellose sodium, and
C) hydroxypropyl cellulose,
the total amount of the low-substituted hydroxypropyl cellulose and croscarmellose sodium is 10-20% by mass.

In one embodiment, the present invention is the following tablet.

A tablet comprising the following A) to C):
A) a granulated material containing the following 1) to 3):
   1) 55 to 65% by mass of Linzagolix or a pharmacologically acceptable salt thereof;
   2) crystalline cellulose; and
   3) low-substituted hydroxypropyl cellulose,
B) croscarmellose sodium, and
C) hydroxypropyl cellulose,
the total amount of the low-substituted hydroxypropyl cellulose and croscarmellose sodium is 10-20% by mass.

In one embodiment, the present invention is the following tablet.

A tablet comprising the following A) and B):
A) a granulated material containing the following 1) to 3):
   1) 15 to 60% by mass of Linzagolix or a pharmacologically acceptable salt thereof;
   2) crystalline cellulose; and
   3) low-substituted hydroxypropyl cellulose; and
B) croscarmellose sodium.

In one embodiment, the present invention is the following tablet.

A tablet comprising the following A) and B):
A) a granulated material containing the following 1) to 3):
   1) 55 to 65% by mass of Linzagolix or a pharmacologically acceptable salt thereof;
   2) crystalline cellulose; and
   3) low-substituted hydroxypropyl cellulose; and
B) croscarmellose sodium.

In one embodiment, the present invention is the following tablet.

A tablet comprising the following A) and B) as a component to be added later:
A) a granulated material containing the following 1) to 3):
   1) Linzagolix or a pharmacologically acceptable salt thereof having an average particle diameter of 1 to 40 µm;
   2) crystalline cellulose; and
   3) low-substituted hydroxypropyl cellulose; and
B) croscarmellose sodium,
the content of the Linzagolix or a pharmacologically acceptable salt thereof is 15-60% by mass.

In one embodiment, the present invention is the following tablet.

A tablet comprising the following 1) to 5):
1) Linzagolix or a pharmacologically acceptable salt thereof;
2) crystalline cellulose;
3) low-substituted hydroxypropyl cellulose;
4) one or more disintegrants selected from the group consisting of carmellose sodium, carmellose calcium and croscarmellose sodium; and
5) one or more coating agents selected from the group consisting of hypromellose and a polyvinyl alcohol-polyethylene glycol graft copolymer.

In one embodiment, the present invention is the following tablet.

A tablet comprising the following 1) to 5):
1) Linzagolix choline salt;
2) crystalline cellulose;
3) low-substituted hydroxypropyl cellulose;
4) croscarmellose sodium; and
5) a polyvinyl alcohol-polyethylene glycol graft copolymer.

### (oral solid preparation)

Examples of the dosage form of the oral solid preparation of the present invention include a tablet (e.g. an uncoated tablet, a film-coated tablet (FC tablet), a sugar-coated tablet), a pill, a granule and a capsule. The oral solid preparation of the present invention is preferably a tablet, more preferably a film-coated tablet.

The oral solid preparation of the present invention can be produced by a conventional method in the pharmaceutical field using a pharmaceutical additive generally used for an oral solid preparation.

As the additive to be used for the oral solid preparation of the present invention, various additives which do not cause a change in formulation with Linzagolix can be used, and for example, a disintegrant, an excipient, a binder, a lubricant, a coating agent, a colorant, a plasticizer, a glossing agent and the like can be appropriately used. However, the present invention is not limited thereto.

Examples of the disintegrant include low-substituted hydroxypropyl cellulose, carmellose sodium, carmellose calcium, and croscarmellose sodium.

Examples of the excipient include crystalline cellulose, lactose, lactose hydrate, fructose, d-mannitol, erythritol, xylitol, maltose, d-sorbitol, and maltitol.

The excipient is preferably lactose hydrate or D-mannitol, more preferably lactose hydrate.

In the present invention, the amount of the excipient other than crystalline cellulose in the oral solid preparation is 0-60% by mass, preferably 0-35% by mass, more preferably 10-35% by mass.

Examples of the binder include hydroxypropyl cellulose, hypromellose, polyvinylpyrrolidone, polyvinyl alcohol, and polyethylene glycol.

The binder is preferably hydroxypropyl cellulose or hypromellose, more preferably hydroxypropyl cellulose.

In the present invention, the content of the binder in the oral solid preparation is, for example, 5% by mass or less, 4% by mass or less, 3% by mass or less, or 2% by mass or less.

In one embodiment, the content of the binder in the oral solid preparation is preferably 5% by mass, 4% by mass, 3% by mass or 2% by mass.

In one embodiment, the content of the binder in the oral solid preparation is preferably 4% by mass or less, more preferably 3% by mass or less, and most preferably 2% by mass or less.

Examples of the lubricant include magnesium stearate, calcium stearate, stearic acid, talc, light silicic anhydride, a sucrose fatty acid ester, sodium stearyl fumarate, polyethylene glycol, and glycerin monostearate.

Examples of the coating agent include hypromellose, hydroxypropyl cellulose, polyvinylpyrrolidone, polyvinyl alcohol, and a polyvinyl alcohol-polyethylene glycol graft copolymer.

The coating agent is preferably hypromellose, hydroxypropyl cellulose, or a polyvinyl alcohol-polyethylene glycol graft copolymer, more preferably hypromellose or a polyvinyl alcohol-polyethylene glycol graft copolymer, and most preferably a a polyvinyl alcohol-polyethylene glycol graft copolymer. Specific examples thereof include Kollicoat(registered trademark) IR (manufactured by BASF).

Examples of the plasticizer include polyethylene glycol, propylene glycol, triacetin, triethyl citrate, glycerin, glycerin fatty acid ester, and polyethylene glycol.

Examples of the colorant include food colorings such as Food Yellow No. 4, Food Yellow No. 4 Aluminum Lake; Food Yellow No. 5, Food Red No. 2, Food Red No. 3, Food Red No. 102, Food Blue No. 1, Food Blue No. 2, and Food Blue No. 2 Aluminum Lake; titanium oxide; talc; yellow iron oxide; yellow ferric oxide; ferric oxide and brown iron oxide.

The colorant is preferably titanium oxide, talc, yellow ferric oxide or ferric oxide.

Examples of the glossing agent include carnauba wax.

### (Method for producing oral solid preparation)

The oral solid preparation of the present invention can be produced by compression-molding a mixture of Linzagolix or a pharmacologically acceptable salt thereof and additives such as an excipient, a disintegrant, and a binder. For example, there may be mentioned a method for producing a tablet by mixing Linzagolix or a pharmacologically acceptable salt thereof and additives such as an excipient, a disintegrant and a binder in an appropriate mixer, and then, directly tableting the mixture. Alternatively, a method for producing a granulated material for a tablet by dry granulation using a roller compactor, or a method for producing a granulated material for a tablet by wet granulation using water or a solution in which a binder is dissolved in a solvent such as water may be used.

Specifically, the oral solid preparation of the present invention can be produced according to the following production process.

In the present invention, "granulation", "mixing", "sizing", "sieving", "tableting" and "film coating" may be carried out by a conventional method in the pharmaceutical technical field.

The method for producing the oral solid preparation of the present invention comprises the following (a) to (d):
(a) a step of mixing Linzagolix or a pharmacologically acceptable salt thereof, crystalline cellulose, low-substituted hydroxypropyl cellulose and additives.
(b) a step of granulating the mixture obtained in the step (a),
(c) a step of mixing the granulated material obtained in the step (b) with additives, and
(d) a step of tableting the mixture obtained in the step (c).

The method for producing the oral solid preparation of the present invention further includes, if necessary, a film-coating step of film-coating the tablet (uncoated tablet) obtained in the step (d) to produce a film-coated tablet.

### Step (a)

The step (a) is a step of mixing Linzagolix or a pharmacologically acceptable salt thereof, crystalline cellulose, low-substituted hydroxypropyl cellulose and additives.

It can also be carried out by mixing Linzagolix or a pharmacologically acceptable salt thereof, crystalline cellulose, low-substituted hydroxypropyl cellulose and additives.

In one embodiment of the step (a), the additives include, for example, an excipient, a disintegrant, and the like. The additives are preferably one or more disintegrants selected from the group consisting of carmellose sodium, carmellose calcium and croscarmellose sodium, and an excipient such as lactose hydrate or D-mannitol.

The mixing can also be carried out using, for example, a mixer such as a V-type mixer or a Bohle container. The mixing may be carried out using a granulator such as a stirring granulator, a fluidized bed granulator or a dry granulator used in the step (b).

### Step (b)

The step (b) is a step of granulating the mixture obtained in the step (a).

The mixture obtained in the step (a) can also be granulated with a binder (e.g. hydroxypropyl cellulose). If necessary, the resulting granulated material may be subjected to sizing.

The granulation may be performed using, for example, a high-shear granulation method, a fluidized bed granulation method, a dry granulation method, or the like.

For the granulation, for example, a fluidized bed granulator, a tumbling fluidized bed granulator, a high-speed mixing stirring granulator, or the like can also be used.

The sizing may be carried out by either wet sizing or dry sizing.

For the sizing, for example, a sizing machine such as a speed mill, Comil, or Fiore can be used.

For example, the granulation may be carried out by spraying a solution in which a binder is dissolved in a solvent such as water and/or water to the mixture obtained in the step (a), followed by drying and, if necessary, sizing to obtain a granulated material. The granulation in the step (b) is preferably carried out by a stirring granulation method because the content of Linzagolix or a pharmacologically acceptable salt thereof can be increased. In the case of the stirring granulation method, the granulated material may be subjected to wet sizing, if necessary, before drying.

In one embodiment, a granulated material containing Linzagolix or a pharmacologically acceptable salt thereof can also be produced by mixing 15 to 60% by mass of Linzagolix or a pharmacologically acceptable salt thereof, 15 to 30% by mass of crystalline cellulose, and 5 to 10% by mass of low-substituted hydroxypropyl cellulose, and then granulating the resulting mixture while spraying an aqueous solution of a binder and, if necessary, water.

In one embodiment, a granulated material containing Linzagolix or a pharmacologically acceptable salt thereof can also be produced by mixing 15 to 60% by mass of Linzagolix or a pharmacologically acceptable salt thereof, 15 to 30% by mass of crystalline cellulose, 5 to 10% by mass of low-substituted hydroxypropyl cellulose, 1 to 60% by mass of lactose hydrate and 5 to 10% by mass of one or more disintegrants selected from the group consisting of croscarmellose sodium and carmellose calcium, and then granulating the resulting mixture while spraying an aqueous solution of a binder and, if necessary, water.

### Step (c)

The step (c) is a step of mixing the granulated material obtained in the step (b) with additives.

The additives may be added to and mixed with the granulated material obtained in the step (b) to obtain a mixture for tableting.

The mixing can also be performed using a mixer such as a V-type mixer or a Bohle container.

In one embodiment of the step (c), the additives include, for example, a disintegrant, a lubricant, and the like. The additives are preferably one or more disintegrants selected from the group consisting of carmellose sodium, carmellose calcium and croscarmellose sodium, a lubricant such as magnesium stearate, and the like.

In one embodiment, in this step, 5 to 10% by mass of a disintegrant and 1 to 5% by mass of a lubricant may be used.

### Step (d)

The step (d) is a step of tableting the mixture obtained in the step (c).

The tableting (compression molding) can be carried out by tableting the mixture obtained in the step (c) using, for example, a single-punch tableting machine, a rotary tableting machine or the like at a tableting pressure of, for example, 1 to 20 kN, preferably 5 to 15 kN.

### Film coating step

A film-coated tablet can also be obtained by spraying a coating solution on the tablet (uncoated tablet) obtained by the above method.

The film coating method is not particularly limited, and for example, a film-coated tablet can also be produced by placing the obtained tablet (uncoated tablet) in a coating pan and spraying a coating solution.

For example, a film-coated tablet can also be produced by coating the tablet (uncoated tablet) of the present invention while spraying a coating solution containing a coating agent such as hypromellose or a polyvinyl alcohol-polyethylene glycol graft copolymer (if necessary, a plasticizer, a colorant or the like may be added to the coating solution). The amount of the coating agent used for film coating is usually 1 to 20 parts by mass, preferably 2 to 15 parts by mass, more preferably 2 to 10 parts by mass with respect to 100 parts by mass of the tablet (uncoated tablet).

The coating agent may be used in combination with a plasticizer, a colorant, a lubricant, or the like.

If necessary, after the film coating, a glossing agent such as carnauba wax may be added for polishing.

The polishing may be carried out by a conventional method, and for example, a glossing agent such as carnauba wax may be added to polish the film-coated tablet.

The compounded amounts of the coating agent, plasticizer, lubricant and colorant in the above-mentioned film coating may be in accordance with conventional compounded amounts.

The excipient, disintegrant, binder, lubricant and the like used in the production method of the present invention are the same as those mentioned in the present specification.

Hereinafter, the method for producing the oral solid preparation of the present invention will be exemplified, but the method is not limited thereto.

### [Production Example 1 of Tablet (Uncoated Tablet)]

For example, a granulated material containing Linzagolix or a pharmacologically acceptable salt thereof can also be produced by mixing Linzagolix or a pharmacologically acceptable salt thereof, crystalline cellulose, and low-substituted hydroxypropyl cellulose using a mixer, and then granulating the mixture while spraying an aqueous solution of a binder (hydroxypropyl cellulose) and, if necessary, water. The mixing and granulation step may be performed by a high-speed mixing and stirring granulation method, a tumbling fluidized bed granulation method, a fluidized bed granulation method, or the like, and is preferably performed by a high-speed mixing and stirring granulation method.

Subsequently, a tablet (uncoated tablet) can also be produced by adding one or more disintegrants selected from the group consisting of carmellose sodium, carmellose calcium and croscarmellose sodium to the obtained granulated material, and mixing using a mixer, and further adding and mixing one or more lubricants selected from sodium stearyl fumarate, calcium stearate, magnesium stearate, talc and light silicic anhydride, and then tableting the mixture. In the mixing step, an excipient or the like may be further added as necessary.

### [Production Example 2 of Tablet (Uncoated Tablet)]

For example, a granulated material containing Linzagolix or a pharmacologically acceptable salt thereof can also be produced by mixing Linzagolix or a pharmacologically acceptable salt thereof; crystalline cellulose; low-substituted hydroxypropyl cellulose; and one or more disintegrants selected from the group consisting of carmellose sodium, carmellose calcium and croscarmellose sodium using a mixer, and then granulating the mixture while spraying an aqueous solution of a binder (hydroxypropyl cellulose) and, if necessary, water. The mixing and granulation step may be performed by a high-speed mixing and stirring granulation method, a tumbling fluidized bed granulation method, a fluidized bed granulation method, or the like, and is preferably performed by a high-speed mixing and stirring granulation method.

Subsequently, a tablet (uncoated tablet) can also be produced by adding the obtained granulated material and one or more lubricants selected from sodium stearyl fumarate, calcium stearate, magnesium stearate, talc and light silicic anhydride, and mixing them using a mixer, and then tableting the mixture. In the mixing step, if necessary, one or more additives may be further added. Examples of the additive include an excipient and the like.

### [Production example of film-coated tablet]

For example, a film-coated tablet can also be produced by coating the tablet (uncoated tablet) of the present invention while spraying a coating solution containing a coating agent such as hypromellose or a polyvinyl alcohol-polyethylene glycol graft copolymer (if necessary, a plasticizer, a colorant or the like may be added to the coating solution).

After the film coating, a glossing agent such as carnauba wax may be added to perform polishing as necessary.

The oral solid preparation of the present invention preferably has an appropriate hardness from the viewpoint of convenience in production and transportation. For example, in a hardness test, in the case of an uncoated tablet having a tablet mass of 200 mg, the hardness may be usually adjusted to80 N or more, preferably 100 N or more, and more preferably 150 N or more.

The hardness can also be adjusted by appropriately selecting the kind or amount of a pharmaceutical additive, a production method (for example, a granulation method or the like), a production condition (for example, a tableting pressure or the like) or the like.

The oral solid preparation of the present invention, e.g., a tablet or a capsule, has a size that allows good swallowing. For example, the longest dimension of an oval tablet or a capsule is 20 mm or less and the diameter of a circular tablet is 13 mm or less.

The tablet of the present invention has a hardness of, for example, 80 N or more, preferably 100 N or more, and more preferably 150 N or more.

In the oral solid preparation of the present invention, for example, in the case of a tablet, examples of the mass per tablet include 50 to 1000 mg; 50 to 800 mg; 50 to 500 mg; 50 to 300 mg; 100 to 300 mg, and 100 to 200 mg.

When the oral solid preparation of the present invention is used for actual therapy, the dosage of Linzagolix or a pharmacologically acceptable salt thereof is appropriately determined according to the sex, age and body weight of the patient, the severity of the disease and the like, but it can be administered in the range of about 0.1 mg to 1000 mg in terms of Linzagolix per day for an adult. Preferably, a dose of 25 mg to 200 mg in terms of Linzagolix is orally administered to an adult per day in 1 to 3 divided doses a day.

### Examples

The contents of the present invention will be described in more detail with reference to the following Test Examples, Examples, and Comparative Examples, but the contents of the present invention are not limited thereto.

### Reference Example 1

Linzagolix choline salt (2406 mg), D-mannitol (Mannit P, manufactured by Mitsubishi Shoji Food Tech Co., Ltd.) (114 mg), crystalline cellulose (Ceolus (registered trademark) PH-101, manufactured by Asahi Kasei Chemicals Corporation) (520 mg), and low-substituted hydroxypropyl cellulose (LH-11, manufactured by Shin-Etsu Chemical Co., Ltd.) (800 mg) were mixed in a mortar. To the resulting mixture, a binding liquid (6% aqueous hydroxypropyl cellulose solution) prepared by dissolving 80 mg of hydroxypropyl cellulose (HPC-L, manufactured by Nippon Soda Co., Ltd.) in 1253 mg of purified water was added, and 1017 mg of purified water was further added thereto to effect granulation. The obtained granulated material was dried at 60°C for 3 hours, and the dried product was sized with a sieve opening of 500 µm. To 3716 mg of the sized granulated material, 75.8 mg of magnesium stearate (manufactured by Taihei Chemical Industrial Co., Ltd.) was added and mixed. The obtained mixture was tableted using a single-punch tableting machine (N-30E, manufactured by Okada Seiko Co., Ltd.) with a punch and die diameter of 6 mm at a tableting pressure of about 5 kN to give a tablet (uncoated tablet) with a mass of 100 mg containing 60.15 mg of Linzagolix choline salt (50 mg in terms of Linzagolix) per tablet.

The dissolution property of the tablet prepared in Reference Example 1 was evaluated by the dissolution test described in Test Example 1.

The formulation of the tablet prepared in Reference Example 1 and the result of the dissolution test are summarized in Table 1.

### Summarized.

**[Table 1]**

| Component | Reference Example 1 | |
|---|---|---|
| | Amount per tablet (mg) | Content (%) |
| Linzagolix choline salt (Linzagolix) | 60.15 (50) | 60.2 |
| D-mannitol | 2.85 | 2.85 |
| crystalline cellulose | 13 | 13 |
| low-substituted hydroxypropyl cellulose | 20 | 20 |
| hydroxypropyl cellulose | 2 | 2 |
| magnesium stearate | 2 | 2 |
| total | 100 | |
| dissolution ratio in 15 minutes (%) | 38.2 | |

### Example 1

(1) Linzagolix choline salt (481.2 g), 502.8 g of lactose hydrate (Pharmatose (registered trademark) 200M, manufactured by DFE Pharma), 400 g of crystalline cellulose (Ceolus (registered trademark) PH-101, manufactured by Asahi Kasei Chemicals Corporation), and 80 g of low-substituted hydroxypropyl cellulose (LH-21, manufactured by Shin-Etsu Chemical Co., Ltd.) were mixed using a high-speed mixing stirring granulator (FM-VG-10, manufactured by Powrex Corporation). To this mixture, a binding liquid (7% aqueous hydroxypropyl cellulose solution) prepared by dissolving 32 g of hydroxypropyl cellulose (HPC-L, manufactured by Nippon Soda Co., Ltd.) in 425 g of purified water was sprayed with a spray nozzle, and granulation was performed while further spraying 171 g of purified water with a spray nozzle. The obtained granulated material was dried using a fluidized bed dryer (FD-LAB-1, manufactured by Powrex Corporation), and then sized using a sizing machine (Comil QC-197S, manufactured by Powrex Corporation) with a screen size of φ 1.143 mm.
(2) Using a V-type mixer (DV-1, manufactured by Dalton Co., Ltd.), 1420.4 g of the obtained sized granulated material and 74.8 g of croscarmellose sodium (Ac-Di-Sol SD-711, manufactured by FMC Corporation) were mixed, and further magnesium stearate (manufactured by Merck Millipore Corporation) was added and mixed. The obtained mixture was tableted by a rotary tableting machine (CLEANPRESS Correct 12HUK, manufactured by Kikusui Seisakusho Ltd.) under the conditions of a punch and die diameter of 8 mm and a tableting pressure of about 10 kN to give a tablet (uncoated tablet) with a mass of 200 mg containing 60.15 mg of Linzagolix choline salt (50 mg in terms of Linzagolix) per tablet.
(3) Titanium oxide (manufactured by Freund Corporation) (9.36 g), 0.72 g of yellow ferric oxide (manufactured by Kishi Kasei Co., Ltd.) and 14.4 g of talc (manufactured by Matsumura Sangyo Co., Ltd.) were dispersed in 288 g of purified water. To the obtained dispersion liquid, 47.52 g of Kollicoat (registered trademark) IR (manufactured by BASF) was added and dissolved. The mixture was passed through a 150 µm sieve to obtain a coating solution.
(4) Using a tablet coating machine (HCT-MINI, manufactured by Freund Corporation), the tablet (uncoated tablet) obtained in the above (2) was coated with the coating solution obtained in the above (3) so that the coating amount per tablet was 8 mg, to give a film-coated tablet.

### Example 2

(1) OPADRY YELLOW (containing hypromellose 2910, titanium dioxide, macrogol 400, yellow ferric oxide and ferric oxide) (manufactured by Colorcon Co., Ltd.) (27 g) was dispersed in 218.5 g of purified water. The mixture was passed through a 150 µm sieve to obtain a coating solution.
(2) Using a tablet coating machine (HCT-MINI, manufactured by Freund Corporation), the tablet (uncoated tablet) obtained in the above Example 1 was coated with the coating solution obtained in the above (1) so that the coating amount per tablet was 6 mg. The obtained tablet was polished by adding carnauba wax (manufactured by Nippon Wax Co., Ltd.) in an amount of 0.02% with respect to the mass of the uncoated tablet to give a film-coated tablet.

### Example 3

(1) Linzagolix choline salt (D50 6.3 µm) (451.125 g), 471.375 g of lactose hydrate (Pharmatose (registered trademark) 200M, manufactured by DFE Pharma), 375 g of crystalline cellulose (Ceolus (registered trademark) PH-101, manufactured by Asahi Kasei Chemicals Corporation), and 75 g of low-substituted hydroxypropyl cellulose (LH-21, manufactured by Shin-Etsu Chemical Co., Ltd.) were mixed using a high-speed mixing stirring granulator (FM-VG-01, manufactured by Powrex Corporation). The mixture was granulated while spraying a binding liquid (5.2% aqueous hydroxypropyl cellulose solution) prepared by dissolving 30 g of hydroxypropyl cellulose (HPC-L, manufactured by Nippon Soda Co., Ltd.) in 545.6 g of purified water with a spray nozzle. The obtained granulated material was subjected to wet sizing using a sizing machine (QC-197S, manufactured by Powrex Corporation) with a screen size of 6.35 mm and square holes, and then dried using a fluidized bed dryer (FD-LAB-1, manufactured by Powrex Corporation). Subsequently, the granulated material was sized with a screen size of φ 1.143 mm using a sizing machine (Comil QC-197S, manufactured by Powrex Corporation).
(2) The obtained sized granulated material (1333.1 g) and 71.3 g of croscarmellose sodium (Ac-Di-Sol SD-711, manufactured by FMC Corporation) were mixed using a V-type mixer (DV-1, manufactured by Dalton Co., Ltd.), and 21.4 g of magnesium stearate (manufactured by Merck Millipore Corporation) was further added and mixed. The resulting mixture was divided into three portions. One portion of the mixture was tableted using a rotary tableting machine (CLEANPRESS Correct 12HUK, manufactured by Kikusui Seisakusho Ltd.) under the conditions of a punch and die diameter of 10 mm and a tableting pressure of about 15 kN to give a tablet (uncoated tablet) with a mass of 400 mg containing 120.3 mg of Linzagolix choline salt (100 mg in terms of Linzagolix) per tablet.
(3) Titanium oxide (manufactured by Freund Corporation) (18.72 g), 28.8 g of talc (manufactured by Matsumura Sangyo Co., Ltd.), and 1.44 g of yellow ferric oxide (manufactured by Kishi Kasei Co., Ltd.) were dispersed in 816 g of purified water. To this, 95.04 g of Kollicoat (registered trademark) IR (manufactured by BASF) was added and dissolved, and the solution was passed through a 150 µm sieve to obtain a coating solution.
(4) Using a tablet coating machine (HCT-MINI, manufactured by Freund Corporation), the tablet (uncoated tablet) obtained in the above (2) was coated with the coating solution obtained in the above (3) so that the coating amount per tablet was 16 mg. The obtained tablet was polished by adding carnauba wax (manufactured by Nippon Wax Co., Ltd.) in an amount of 0.02% with respect to the mass of the uncoated tablet to give a film-coated tablet.

### Example 4

(1) One portion of the mixture divided in Example 3 was tableted using a rotary tableting machine (CLEANPRESS Correct 12HUK, manufactured by Kikusui Seisakusho Ltd.) under the conditions of a punch and die size of 16.5 × 7.7 mm and a tableting pressure of about 7 kN to give a tablet (uncoated tablet) with a mass of 800 mg containing 240.6 mg of Linzagolix choline salt (200 mg in terms of Linzagolix) per tablet.
(2) Titanium oxide (manufactured by Freund Corporation) (18.72 g), 28.8 g of talc (manufactured by Matsumura Sangyo Co., Ltd.), and 1.44 g of yellow ferric oxide (manufactured by Kishi Kasei Co., Ltd.) were dispersed in 816 g g of purified water. To this, 95.04 g of Kollicoat (registered trademark) IR (manufactured by BASF) was added and dissolved, and the solution was passed through a 150 µm sieve to obtain a coating solution.
(3) Using a tablet coating machine (HCT-MINI, manufactured by Freund Corporation), the tablet (uncoated tablet) obtained in the above (1) was coated with the coating solution obtained in the above (2) so that the coating amount per tablet was 32 mg. The obtained tablet was polished by adding carnauba wax (manufactured by Nippon Wax Co., Ltd.) in an amount of 0.02% with respect to the mass of the uncoated tablet to give a film-coated tablet.

### Example 5

Linzagolix choline salt (2406 mg), 74 mg of lactose hydrate (Pharmatose (registered trademark) 200M, manufactured by DFE Pharma), 600 mg of crystalline cellulose (Ceolus (registered trademark) PH-101, manufactured by Asahi Kasei Chemicals Corporation), and 400 mg of low-substituted hydroxypropyl cellulose (LH-11, manufactured by Shin-Etsu Chemical Co., Ltd.) were mixed in a mortar. To this mixture, a binding liquid (10% aqueous hydroxypropyl cellulose solution) prepared by dissolving 80 mg of hydroxypropyl cellulose (HPC-L, manufactured by Nippon Soda Co., Ltd.) in 720 mg of purified water was added, and 740 mg of purified water was further added thereto to effect granulation. The obtained granulated material was dried at 60°C for 2 hours, and the dried product was sized with a 500 µm mesh.

To the sized granulated material (3390 mg), 381 mg of carmellose calcium (ECG-505, manufactured by Gotoku Chemical Company Ltd.) was added and mixed, and 38 mg of magnesium stearate (manufactured by Taihei Chemical Industrial Co., Ltd.) was further added and mixed. The resulting mixture was tableted using a single-punch tableting machine (N-30E, manufactured by Okada Seiko Co., Ltd.) with a punch and die diameter of 6 mm at a tableting pressure of about 5 kN to give a tablet (uncoated tablet) with a mass of 100 mg containing 60.15 mg of Linzagolix choline salt (50 mg in terms of Linzagolix) per tablet.

### Example 6

Linzagolix choline salt (2406 mg), 74 mg of lactose hydrate (Pharmatose (registered trademark) 200M, manufactured by DFE Pharma), 600 mg of crystalline cellulose (Ceolus (registered trademark) PH-101, manufactured by Asahi Kasei Chemicals Corporation), and 600 mg of low-substituted hydroxypropyl cellulose (LH-11, manufactured by Shin-Etsu Chemical Co., Ltd.) were mixed in a mortar. To this mixture, a binding liquid (10% aqueous hydroxypropyl cellulose solution) prepared by dissolving 80 mg of hydroxypropyl cellulose (HPC-L, manufactured by Nippon Soda Co., Ltd.) in 720 mg of purified water was added, and 790 mg of purified water was further added to effect granulation. The obtained granulated material was dried at 60°C for 2 hours, and the dried product was sized with a 500 µm mesh.

To the sized granulated material (3580 mg), 190 mg of croscarmellose sodium (Ac-Di-Sol SD-711, manufactured by FMC Corporation) was added and mixed, and 38 mg of magnesium stearate (manufactured by Merck Millipore Corporation) was further added and mixed. The obtained mixture was tableted using a single-punch tableting machine (N-30E, manufactured by Okada Seiko Co., Ltd.) under the conditions of a punch and die diameter of 6 mm and a tableting pressure of about 5 kN to give a tablet (uncoated tablet) with a mass of 100 mg containing 60.15 mg of Linzagolix choline salt (50 mg in terms of Linzagolix) per tablet.

### Comparative Example 1

Linzagolix choline salt (2406 mg), 74 mg of lactose hydrate (Pharmatose (registered trademark) 200M, manufactured by DFE Pharma), 600 mg of crystalline cellulose (Ceolus (registered trademark) PH-101, manufactured by Asahi Kasei Chemicals Corporation), and 600 mg of low-substituted hydroxypropyl cellulose (LH-11, manufactured by Shin-Etsu Chemical Co., Ltd.) were mixed in a mortar. To this mixture, a binding liquid (10% aqueous hydroxypropyl cellulose solution) prepared by dissolving 80 mg of hydroxypropyl cellulose (HPC-L, manufactured by Nippon Soda Co., Ltd.) in 720 mg of purified water was added, and 870 mg of purified water was further added to effect granulation. The obtained granulated material was dried at 60°C for 2 hours, and the dried product was sized with a 500 µm mesh.

To the sized granulated material (3571 mg), crospovidone (XL-10) (manufactured by ISP Japan Ltd.) (190 mg) was added and mixed, and magnesium stearate (manufactured by Merck Millipore Corporation) (38 mg) was further added and mixed. The obtained mixture was tableted using a single-punch tableting machine (N-30E, manufactured by Okada Seiko Co., Ltd.) under the conditions of a punch and die diameter of 6 mm and a tableting pressure of about 5 kN to give a tablet (uncoated tablet) with a mass of 100 mg containing 60.15 mg of Linzagolix choline salt (50 mg in terms of Linzagolix) per tablet.

### Comparative Example 2

Linzagolix choline salt (4812 mg), 2148 mg of lactose hydrate (Pharmatose (registered trademark) 200M, manufactured by DFE Pharma) and 400 mg of low-substituted hydroxypropyl cellulose (LH-21, manufactured by Shin-Etsu Chemical Co., Ltd.) were mixed in a mortar. To this mixture, a binding liquid (10% aqueous hydroxypropyl cellulose solution) prepared by dissolving 160 mg of hydroxypropyl cellulose (HPC-L, manufactured by Nippon Soda Co., Ltd.,) in 1440 mg of purified water was added, and 650 mg of purified water was further added to effect granulation. The obtained granulated material was dried at 60°C for 3 hours, and the dried product was sized with a 500 µm mesh.

To the sized granulated material (7196 mg), 383 mg of croscarmellose sodium (Ac-Di-Sol SD-711, manufactured by FMC Corporation) was added and mixed, and further 76 mg magnesium stearate (manufactured by Merck Millipore Corporation) was added and mixed. The obtained mixture was tableted by a single-punch tableting machine (N-30E, manufactured by Okada Seiko Co., Ltd.) under the conditions of a punch and die diameter of 8 mm and a tableting pressure of about 5 kN to give a tablet (uncoated tablet) with a mass of 200 mg containing 120.3 mg of Linzagolix choline salt (100 mg in terms of Linzagolix) per tablet.

### Example 7

Linzagolix choline salt (4812 mg), 2148 mg of crystalline cellulose (Ceolus (registered trademark) PH-101, manufactured by Asahi Kasei Chemicals Corporation), and 400 mg of low-substituted hydroxypropyl cellulose (LH-21, manufactured by Shin-Etsu Chemical Co., Ltd.) were mixed in a mortar. To this mixture, a binding liquid (10% aqueous hydroxypropyl cellulose solution) prepared by dissolving 160 mg of hydroxypropyl cellulose (HPC-L, manufactured by Nippon Soda Co., Ltd.) in 1440 mg of purified water was added, and 2650 mg of purified water was further added to effect granulation. The obtained granulated material was dried at 60°C for 3 hours, and the dried product was sized with a 500 µm mesh.

To the sized granulated material (7240 mg), 385 mg of croscarmellose sodium (Ac-Di-Sol SD-711, manufactured by FMC Corporation) was added and mixed, and further 76 mg magnesium stearate (manufactured by Merck Millipore Corporation) was added and mixed. The obtained mixture was tableted by a single-punch tableting machine (N-30E, manufactured by Okada Seiko Co., Ltd.) under the conditions of a punch and die diameter of 8 mm and a tableting pressure of about 5 kN to give a tablet (uncoated tablet) with a mass of 200 mg containing 120.3 mg of Linzagolix choline salt (100 mg in terms of Linzagolix) per tablet.

### Example 8

Linzagolix choline salt (4812 mg), 788 mg of lactose hydrate (Pharmatose (registered trademark) 200M, manufactured by DFE Pharma), 1200 mg of crystalline cellulose (Ceolus (registered trademark) PH-101, manufactured by Asahi Kasei Chemicals Corporation), and 400 mg of low-substituted hydroxypropyl cellulose (LH-21, manufactured by Shin-Etsu Chemical Co., Ltd.) were mixed in a mortar. To this mixture, a binding liquid (18% aqueous hydroxypropyl cellulose solution) prepared by dissolving 320 mg of hydroxypropyl cellulose (HPC-SL, manufactured by Nippon Soda Co., Ltd.) in 1458 mg of purified water was added, and 1238 mg of purified water was further added to effect granulation. The obtained granulated material was dried at 60°C for 3 hours, and the dried product was sized with a 500 µm mesh.

To the sized granulated material (7156 mg), 381 mg of croscarmellose sodium (Ac-Di-Sol SD-711, manufactured by FMC Corporation) was added and mixed, and further 76 mg magnesium stearate (manufactured by Merck Millipore Corporation) was added and mixed. The obtained mixture was tableted by a single-punch tableting machine (N-30E, manufactured by Okada Seiko Co., Ltd.) under the conditions of a punch and die diameter of 8 mm and a tableting pressure of about 5 kN to give a tablet (uncoated tablet) with a mass of 200 mg containing 120.3 mg of Linzagolix choline salt (100 mg in terms of Linzagolix) per tablet.

### Example 9

(1) Linzagolix choline salt (210.525 g), 797.475 g of lactose hydrate (Pharmatose (registered trademark) 200M, manufactured by DFE Pharma), 210 g of crystalline cellulose (Ceolus (registered trademark) PH-101, manufactured by Asahi Kasei Chemicals Corporation), and 70 g of low-substituted hydroxypropyl cellulose (LH-21, manufactured by Shin-Etsu Chemical Co., Ltd.) were mixed using a high-speed mixing stirring granulator (FM-VG-10, manufactured by Powrex Corporation). To this mixture, a binding liquid (10% aqueous hydroxypropyl cellulose solution) prepared by dissolving 28 g of hydroxypropyl cellulose (HPC-L, manufactured by Nippon Soda Co., Ltd.) in 252 g of purified water was sprayed by a spray nozzle, and granulation was carried out while further spraying 170 g of purified water by a spray nozzle. The obtained granulated material was dried at 80°C using a fluidized bed dryer (FD-LAB-1, manufactured by Powrex Corporation), and the dried product was sized with a 1 mm mesh.
(2) 1260.9 g of the granulated material obtained in the above (1) and 67.1 g of croscarmellose sodium (Ac-Di-Sol SD-711, manufactured by FMC Corporation) were mixed using a V-type mixer (DV-1, manufactured by Dalton Co., Ltd.), and 13.4 g of magnesium stearate (manufactured by Merck Millipore Corporation) was further added and mixed. The obtained mixture was tableted using a rotary tableting machine (CLEANPRESS Correct 12HUK, manufactured by Kikusui Seisakusho Ltd.) under the conditions of a punch and die diameter of 8 mm and a tableting pressure of about 13 kN to give a tablet (uncoated tablet) with a mass of 200 mg containing 30.075 mg of Linzagolix choline salt (25 mg in terms of Linzagolix) per tablet.
(3) Titanium oxide (manufactured by Freund Corporation) (9.36 g), 14.4 g of talc (manufactured by Matsumura Sangyo Co., Ltd.), and 0.72 g of yellow ferric oxide (manufactured by Kishi Kasei Co., Ltd.) were dispersed in 288 g of purified water. To this, 47.52 g of Kollicoat (registered trademark) IR (manufactured by BASF) was added and dissolved, and the solution was passed through a 150 µm sieve to obtain a coating solution.
(4) Using a tablet coating machine (HCT-MINI, manufactured by Freund Corporation), the tablet (uncoated tablet) obtained in the above (2) was coated with the coating solution obtained in the above (3) so that the coating amount per tablet was 8 mg, to give a film-coated tablet.

### Example 10

(1) Linzagolix choline salt (842.1 g), 165.9 g of lactose hydrate (Pharmatose (registered trademark) 200M, manufactured by DFE Pharma), 210 g of crystalline cellulose (Ceolus (registered trademark) PH-101, manufactured by Asahi Kasei Chemicals Corporation), and 70 g of low-substituted hydroxypropyl cellulose (LH-21, manufactured by Shin-Etsu Chemical Co., Ltd.) were mixed using a high-speed mixing stirring granulator (FM-VG-10, manufactured by Powrex Corporation). To this mixture, a binding liquid (10% aqueous hydroxypropyl cellulose solution) prepared by dissolving 28 g of hydroxypropyl cellulose (manufactured by Nippon Soda Co., Ltd.) in 252 g of purified water was sprayed by a spray nozzle, and granulation was carried out while further spraying 250 g of purified water by a spray nozzle. The obtained granulated material was dried at 80°C using a fluidized bed dryer (FD-LAB-1, manufactured by Powrex Corporation), and the dried product was sized with a 1 mm mesh.
(2) Using a V-type mixer (DV-1, manufactured by Dalton Co., Ltd.), 1256.6 g of the granulated material obtained in the above (1) and 66.8 g of croscarmellose sodium (Ac-Di-Sol SD-711, manufactured by FMC Corporation) were mixed, and 13.4 g of magnesium stearate (manufactured by Merck Millipore Corporation) was further added and mixed. The obtained mixture was tableted by a rotary tableting machine (CLEANPRESS Correct 12HUK, manufactured by Kikusui Seisakusho Ltd.) under the conditions of a punch and die diameter of 8 mm and a tableting pressure of about 13 kN to give a tablet (uncoated tablet) with a mass of 200 mg containing 120.3 mg of Linzagolix choline salt (100 mg in terms of Linzagolix) per tablet.
(3) Using a tablet coating machine (HCT-MINI, manufactured by Freund Corporation), the tablet (uncoated tablet) obtained in the above (2) was coated with the coating solution obtained in the above Example 9 (3) so that the coating amount per tablet was 8 mg, to give a film-coated tablet.

### Example 11

A tablet having the composition shown in Table 5 was prepared according to the method of Example 3 using Linzagolix choline salt having a D50 of 33.6 µm.

### [Test Example 1]

### Dissolution test 1

The tablets prepared in Reference Example 1, Comparative Examples 1 and 2, and Examples 1, 2, and 5 to 10 were subjected to a dissolution test according to the dissolution test method paddle method described in the Japanese Pharmacopoeia Sixteenth Edition at a paddle rotation speed of 50 rpm using 900 mL of purified water as a test solution, the sample absorbance of the Linzagolix in the sampling solution was measured by an ultraviolet spectrophotometer (flow-cell method) to determine the dissolution ratio. The results are shown in Tables 1 to 5.
detector: ultraviolet absorptiometer (measurement wavelength: 225 nm)

### [Test Example 2]

### Dissolution test 2

The tablets prepared in Examples 3, 4 and 11 were subjected to a dissolution test in accordance with the dissolution test method paddle method described in the Japanese Pharmacopoeia Seventeenth Edition at a paddle rotation speed of 50 rpm using Japanese Pharmacopoeia Dissolution Test Solution 2 (pH 6.8) as a test solution, and Linzagolix in the sampling solution was quantified by high performance liquid chromatography to determine the dissolution ratio.
detector: ultraviolet absorptiometer (measurement wavelength: 275 nm)

The formulations of the tablets prepared in Examples 1 to 4 and the results of the dissolution test are summarized in Table 2.

**[Table 2]**

| Component | Example 1 | | Example 2 | | Example 3 | | Example 4 | |
|---|---|---|---|---|---|---|---|---|
| | Amount per tablet (mg) | Content (%) | Amount per tablet (mg) | Content (%) | Amount per tablet (mg) | Content (%) | Amount per tablet (mg) | Content (%) |
| Linzagolix choline salt (Linzagolix) | 60.15 (50) | 30.1 | 60.15 (50) | 30.1 | 120.3 (100) | 30.1 | 240.6 (200) | 30.1 |
| lactose hydrate | 62.85 | 31.4 | 62.85 | 31.4 | 125.7 | 31.4 | 251.4 | 31.4 |
| crystalline cellulose | 50 | 25 | 50 | 25 | 100 | 25 | 200 | 25 |
| low-substituted hydroxypropyl cellulose | 10 | 5 | 10 | 5 | 20 | 5 | 40 | 5 |
| croscarmellose sodium | 10 | 5 | 10 | 5 | 20 | 5 | 40 | 5 |
| hydroxypropyl cellulose | 4 | 2 | 4 | 2 | 8 | 2 | 8 | 2 |
| magnesium stearate | 3 | 1.5 | 3 | 1.5 | 6 | 1.5 | 6 | 1.5 |
| subtotal | 200 | | 200 | | 400 | | 800 | |
| OPADRY YELLOW | | | 6 | | | | | |
| polyvinyl alcohol-polyethylene glycol graft copolymer | 5.28 | | | | 10.56 | | 21.12 | |
| talc | 1.6 | | | | 3.2 | | 6.4 | |
| titanium oxide | 1.04 | | | | 2.08 | | 1.4 | |
| yellow ferric oxide | Trace (0.08) | | | | Trace (0.16) | | Trace (0.32) | |
| carnauba wax | | | Trace (0.04) | | Trace (0.08) | | Trace (0.16) | |
| total | 208 | | 206.04 | | 416.08 | | 832.16 | |
| uncoated tablet dissolution ratio in 15 minutes (%) | 99.1 | | 99.1 | | | | | |
| FC tablet dissolution ratio in 15 minutes (%) | 99.1 | | 97.5 | | 94.2 (pH 6.8 buffer) | | 92.2 (pH 6.8 buffer) | |

The formulations of the tablets prepared in Examples 5 and 6 and Comparative Example 1 and the results of the dissolution test are summarized in Table 3.

**[Table 3]**

| Component | Example 5 | | Example 6 | | Comparative Example 1 | |
|---|---|---|---|---|---|---|
| | Amount per tablet (mg) | Content (%) | Amount per tablet (mg) | Content (%) | Amount per tablet (mg) | Content (%) |
| Linzagolix choline salt (Linzagolix) | 60.15 (50) | 60.15 | 60.15 (50) | 60.15 | 60.15 (50) | 60.15 |
| lactose hydrate | 1.85 | 1.85 | 1.85 | 1.85 | 1.85 | 1.85 |
| crystalline cellulose | 15 | 15 | 15 | 15 | 15 | 15 |
| low-substituted hydroxypropyl cellulose | 10 | 10 | 15 | 15 | 15 | 15 |
| croscarmellose sodium | | | 5 | 5 | | |
| carmellose calcium | 10 | 10 | | | | |
| crospovidone | | | | | 5 | 5 |
| hydroxypropyl cellulose | 2 | 2 | 2 | 2 | 2 | 2 |
| magnesium stearate | 1 | 1 | 1 | 1 | 1 | 1 |
| total | 100 | | 100 | | 100 | |
| dissolution ratio in 15 minutes (%) | 97.8 | | 99.4 | | 60.4 | |

The formulations of the tablets prepared in Comparative Example 2 and Examples 7 and 8 and the results of the dissolution test are summarized in Table 4.

**[Table 4]**

| Component | Comparative Example 2 | | Example 7 | | Example 8 | |
|---|---|---|---|---|---|---|
| | Amount per tablet (mg) | Content (%) | Amount per tablet (mg) | Content (%) | Amount per tablet (mg) | Content (%) |
| Linzagolix choline salt (Linzagolix) | 120.3 (100) | 60.15 | 120.3 (100) | 60.15 | 120.3 (100) | 60.15 |
| lactose hydrate | 53.7 | 26.85 | | | 19.7 | 9.85 |
| crystalline cellulose | | | 53.7 | 26.85 | 30 | 15 |
| low-substituted hydroxypropyl cellulose | 10 | 5 | 10 | 5 | 10 | 5 |
| croscarmellose sodium | 10 | 5 | 10 | 5 | 10 | 5 |
| hydroxypropyl cellulose | 4 | 2 | 4 | 2 | 8 | 4 |
| magnesium stearate | 2 | 1 | 2 | 1 | 2 | 1 |
| total | 200 | | 200 | | 200 | |
| dissolution ratio in 15 minutes (%) | 74.1 | | 100.0 | | 91.2 | |

The formulations of the tablets prepared in Examples 9 to 11 and the results of the dissolution test are summarized in Table 5.

**[Table 5]**

| Component | Example 9 | | Example 10 | | Example 11 | |
|---|---|---|---|---|---|---|
| | Amount per tablet (mg) | Content (%) | Amount per tablet (mg) | Content (%) | Amount per tablet (mg) | Content (%) |
| Linzagolix choline salt (Linzagolix) | 30.075 (25) | 15.04 | 120.3 (100) | 60.15 | 120.3 (100) | 30.1 |
| lactose hydrate | 113.925 | 56.96 | 23.7 | 11.85 | 125.7 | 31.4 |
| crystalline cellulose | 30 | 15 | 30 | 15 | 100 | 25 |
| low-substituted hydroxypropyl cellulose | 10 | 5 | 10 | 5 | 20 | 5 |
| croscarmellose sodium | 10 | 5 | 10 | 5 | 20 | 5 |
| hydroxypropyl cellulose | 4 | 2 | 4 | 2 | 8 | 2 |
| magnesium stearate | 2 | 1 | 2 | 1 | 6 | 1.5 |
| subtotal | 200 | | 200 | | 400 | |
| polyvinyl alcohol-polyethylene glycol graft copolymer | 5.28 | | 5.28 | | 10.56 | |
| talc | 1.6 | | 1.6 | | 3.2 | |
| titanium oxide | 1.04 | | 1.04 | | 2.08 | |
| yellow ferric oxide | Trace (0.08) | | Trace (0.08) | | Trace (0.16) | |
| total | 208 | | 208 | | 416.08 | |
| uncoated tablet dissolution ratio in 15 minutes (%) | 104.1 | | 82.1 | | | |
| FC tablet dissolution ratio in 15 minutes (%) | 104.9 | | 88.3 | | 84.2 (pH 6.8 buffer) | |

As shown in Tables 2 to 5, the oral solid preparation of the present invention had a rapid dissolution property. In addition, from the results of Examples 1 and 2, the presence or absence of film coating did not affect the dissolution property.

From the results of Examples 3 and 11, the oral solid preparation of the present invention had a rapid dissolution property even in different particle diameters.

### [Test Example 3]

### Storage stability test

The uncoated tablets and the film-coated tablets (FC tablets) obtained in Examples 9 and 10 were subjected to a storage stability test.

The uncoated tablets and the FC tablets obtained in Examples 9 and 10 were stored under open conditions at a temperature of 40°C and a relative humidity of 75% for 6 months. The content of total analogs was measured by HPLC at the start of storage of the sample and after storage for 1 month, 3 months and 6 months. The results are shown in Table 6.

### Quantification method for analogs

Four tablets of each of the preparations of Example 9 and five tablets of each of the preparations of Example 10 were weighed, and sample solutions were prepared so as to have a concentration of 0.5 mg/mL in terms of Linzagolix. The test was performed by liquid chromatography, and the respective peak areas were measured by an automatic integration method to determine the content (%) of total analogs.
detector: ultraviolet absorptiometer (measurement wavelength: 225 nm)

**[Table 6]**

| | Tablet form | Storage period | | | |
|---|---|---|---|---|---|
| | | at start | 1 month | 3 months | 6 months |
| Example 9 | uncoated tablet | 0.12% | 0.12% | 0.10% | 0.11% |
| | FC tablet | 0.14% | 0.13% | 0.12% | 0.12% |
| Example 10 | uncoated tablet | 0.12% | 0.11% | 0.10% | 0.11% |
| | FC tablet | 0.11% | 0.13% | 0.12% | 0.11% |

From the results shown in Table 6, it was found that the uncoated tablets and the film-coated tablets obtained in Examples 9 and 10 did not show an increase in total analogs with time even after storage for 6 months under open conditions at a temperature of 40°C and a relative humidity of 75%, and thus were stable preparations.

Therefore, it was found that the oral solid preparation of the present invention has a rapid dissolution property even when the content of the active ingredient varies in a wide range, and has good storage stability.

### Industrial Applicability

According to the present invention, it is possible to provide an oral solid preparation having a rapid dissolution property even when the content of Linzagolix or a pharmacologically acceptable salt thereof as an active ingredient varies over a wide range.

## Claims

1. An oral solid preparation comprising the following 1) to 4):
1) Linzagolix or a pharmacologically acceptable salt thereof;
2) crystalline cellulose;
3) low-substituted hydroxypropyl cellulose; and
4) one or more disintegrants selected from the group consisting of carmellose sodium, carmellose calcium and croscarmellose sodium.

2. The oral solid preparation according to claim 1, comprising the following A) and B):
A) a granulated material containing the following 1) to 3):
1) Linzagolix or a pharmacologically acceptable salt thereof;
2) crystalline cellulose; and
3) low-substituted hydroxypropyl cellulose; and
B) one or more disintegrants selected from the group consisting of carmellose sodium, carmellose calcium and croscarmellose sodium.

3. The oral solid preparation according to claim 1 or 2, wherein Linzagolix or a pharmacologically acceptable salt thereof is Linzagolix choline salt.

4. The oral solid preparation according to claim 1 or 2, wherein the dissolution ratio of the oral solid preparation in a pH 6.8 test solution or water after 15 minutes is 80% or more.

5. The oral solid preparation according to claim 1 or 2, wherein the total amount of the low-substituted hydroxypropyl cellulose and one or more disintegrants selected from the group consisting of carmellose sodium, carmellose calcium and croscarmellose sodium in the oral solid preparation is 20% by mass or less.

6. The oral solid preparation according to claim 1 or 2, which comprises a binder.

7. The oral solid preparation according to claim 6, wherein the binder is one or more selected from the group consisting of hydroxypropyl cellulose, hypromellose, polyvinylpyrrolidone, polyvinyl alcohol and polyethylene glycol.

8. The oral solid preparation according to claim 6, wherein the content of the binder in the oral solid preparation is 5% by mass or less.

9. The oral solid preparation according to claim 1 or 2, wherein the content of Linzagolix or a pharmacologically acceptable salt thereof in the oral solid preparation is 15 to 65% by mass.

10. The oral solid preparation according to claim 1 or 2, wherein the content of crystalline cellulose in the oral solid preparation is 15% by mass or more.

11. The oral solid preparation according to claim 1 or 2, which comprises an excipient.

12. The oral solid preparation according to claim 11, wherein the excipient is one or more selected from the group consisting of lactose hydrate and D-mannitol.

13. The oral solid preparation according to claim 1 or 2, wherein the amount of Linzagolix or a pharmacologically acceptable salt thereof per oral solid preparation is 75 mg, 100 mg or 200 mg in terms of Linzagolix.

14. The oral solid preparation according to claim 1 or 2, wherein the oral solid preparation is a tablet.

15. The oral solid preparation according to claim 14, wherein the tablet is a film-coated tablet.

16. The oral solid preparation according to claim 15, wherein the film-coated tablet is a film-coated tablet film-coated with one or more coating agents selected from the group consisting of hypromellose and a polyvinyl alcohol-polyethylene glycol graft copolymer.
